# EUROPEAN PATENT SPECIFICATION

(11) **EP 4 237 064 B1**
(45) Date of publication and mention of the grant of the patent: **21.01.2026**
(21) Application number: 21810841.3
(22) Date of filing: 27.10.2021
(51) Int. Cl.: A61M 25/10, A61F 2/958, A61F 2/24

(54) **PROTECTED PRESSURE-SAFE BALLOONS**
GESCHÜTZTE DRUCKSICHERE BALLONS
BALLONNETS PROTÉGÉS CONTRE LA PRESSION

(30) Priority: 28.10.2020 US 202063106842 P
(43) Date of publication of application: 06.09.2023
(73) Proprietor: Edwards Lifesciences Corporation, Irvine, CA 92614-5688 (US)
(72) Inventor: ZHU, Yidong, M., Irvine, CA 92614 (US)
(74) Representative: Eisenführ Speiser
(86) International application number: PCT/US2021/056760
(87) International publication number: WO 2022/093900

(56) References cited:
- US-A1- 2002 091 435
- US-A1- 2003 028 211
- US-A1- 2015 306 361

## Description

### CROSS-REFERENCE TO RELATED APPLICATIONS

This application claims the benefit of U.S. Provisional Application No. 63/106,842, filed October 28, 2020.

### BACKGROUND OF THE INVENTION

A variety of maladies may affect an individual's body. Such maladies may be of the individual's heart, and may include maladies of the individual's heart valves, including the aortic, mitral, tricuspid, and pulmonary valves. Stenosis, for example, is a common and serious valve disease that may affect the operation of the heart valves and an individual's overall well-being.

Implants may be provided that may replace or repair portions of a patient's heart. Prosthetic implants, such as prosthetic heart valves, may be provided to replace a portion of a patient's heart. Prosthetic aortic, mitral, tricuspid, and even pulmonary valves may be provided.

Implants may be deployed to the desired portion of the patient's body percutaneously, in a minimally invasive manner. Such deployment may occur transcatheter, in which a catheter may be deployed through the vasculature of an individual.

During deployment of such implants, the implants must be expanded to provide an expanded configuration for such implant. Care must be taken to properly expand the implants to a desired implantation site, and to avoid over expansion or under expansion of such implants.

US 2015/0306361 A1 discloses a balloon catheter including a shaft and an inflatable balloon attached to the shaft. The catheter comprises an open sleeve having a proximal end sealingly attached to the catheter shaft and an open distal end. The sleeve surrounds at least part of the balloon. The sleeve and the balloon are arranged such that inflating the balloon expands the sleeve into an expanded state and deflating the balloon when the sleeve is in the expanded state forms an open cavity between the sleeve and the deflated balloon and creates suction to capture and retain debris within the cavity.

### SUMMARY

Expandable implants, or passages or spaces, within a patient's body may be dilated or expanded by inflatable bodies, which may comprise balloons or another form of inflatable body. A system for dilation within a patient's body is defined in claim 1. Preferred embodiments thereof are described in claims 2 through 11. A delivery system for an expandable implant is defined in claim 12 wherein preferred embodiments thereof are described in claims 13 through 15.

Embodiments disclosed herein may be directed to improved expansion or dilation. Embodiments as disclosed herein may include an inflatable device. The inflatable device may include an interior inflatable body configured to inflate in response to receiving fluid. The inflatable device may also include an exterior inflatable body at least partially surrounding the interior inflatable body and configured to inflate when the interior inflatable body inflates and apply an expansion force to the surface to dilate the surface, the exterior inflatable body having a distal portion, a proximal portion, and one or more openings at the distal portion configured to allow the fluid within the interior inflatable body to escape the exterior inflatable body when the interior inflatable body is punctured or bursts.

The exterior inflatable body may shield the interior inflatable body from puncture and may provide support during inflation. The exterior inflatable body may also retain one or more pieces or fragments of the interior inflatable body in the event that the interior inflatable body is punctured or bursts.

Embodiments as disclosed herein may include a delivery system for an expandable implant. The delivery system may include a delivery apparatus configured to deliver the expandable implant to a location in a patient's body. The delivery apparatus may include an elongate shaft and an inflatable device coupled to the elongate shaft. The inflatable device may include an interior inflatable body configured to inflate in response to receiving fluid. The delivery apparatus may also include an exterior inflatable body at least partially surrounding the interior inflatable body and configured to inflate when the interior inflatable body inflates and apply an expansion force to the expandable implant to expand the expandable implant, the exterior inflatable body having a distal portion, a proximal portion, and one or more openings at the distal portion configured to allow the fluid within the interior inflatable body to escape the exterior inflatable body when the interior inflatable body is punctured or bursts.

A method which is not claimed may be for dilation of a surface within a patient's body. The method may include inflating an interior inflatable body of an inflatable device. The method may include inflating an exterior inflatable body of the inflatable device, the exterior inflatable body at least partially surrounding the interior inflatable body and the exterior inflatable body inflating when the interior inflatable body inflates. The exterior inflatable body may have a distal portion, a proximal portion, and one or more openings at the distal portion configured to allow the fluid within the interior inflatable body to escape the exterior inflatable body when the interior inflatable body is punctured or bursts. The method may include applying, by the inflatable device, an expansion force to the surface to dilate the surface.

The method may include allowing, by the exterior inflatable body, fluid within the interior inflatable body to escape the exterior inflatable body when the interior inflatable body is punctured or bursts via the one or more openings at the distal portion of the exterior inflatable body.

### BRIEF DESCRIPTION OF THE DRAWINGS

These and other features, aspects, and advantages are described below with reference to the drawings, which are intended to illustrate, but not to limit, the disclosure. In the drawings, like reference characters denote corresponding features consistently throughout similar embodiments.
FIG. 1 is a cross sectional view of a system for expansion of an expandable implant according to an embodiment of the present disclosure.
FIG. 2A is a cross sectional view of the system shown in FIG. 1, with an inflatable device partially inflated.
FIG. 2B is a graph illustrating compliance of the inflatable device of FIG. 2A, with pressure and the outer diameter of the inflatable device.
FIG. 3A is a cross sectional view of the system shown in FIG. 1, with an inflatable device fully inflated.
FIG. 3B is a graph illustrating compliance of the inflatable device of FIG. 3A, with pressure and the outer diameter of the inflatable device.
FIG. 4 is a cross sectional view of the system shown in FIG. 1, with the interior inflatable body being ruptured.
FIG. 5 is a cross sectional view of a system for expansion of an expandable implant having a plurality of openings, according to another embodiment of the present disclosure.
FIGS. 6A and 6B show a cross sectional view of a system for expansion of an expandable implant having an outer inflatable body with a length that is half the length of the inner inflatable body, according to another embodiment of the present disclosure.
FIGS. 7A and 7B show a cross sectional view of a system for expansion of an expandable implant having an outer inflatable body with a length that is a quarter of the length of the inner inflatable body, according to another embodiment of the present disclosure.
FIG. 8A is a cross sectional view of a system for expansion of an expandable implant according to an embodiment of the present disclosure.
FIG. 8B is a cross sectional view of an exterior inflatable body, according to an embodiment of the present disclosure.
FIG. 8C is a cross sectional view of the exterior inflatable body of FIG. 8A, according to another embodiment of the present disclosure.
FIG. 9 is a graph comparing inflation of a proximal portion of an inflatable device and a distal portion of an inflatable device, according to embodiments of the present disclosure.
FIGS. 10A-10C are cross sectional views of a system for dilating a body or surface within the patient's body.
FIG. 11 is a side view of a delivery apparatus according to an embodiment of the present disclosure.
FIG. 12 illustrates a side view of an implant according to an embodiment of the present disclosure.
FIG. 13 illustrates a perspective view of an implant according to an embodiment of the present disclosure.
FIG. 14 illustrates a perspective view of an implant according to an embodiment of the present disclosure.
FIG. 15 illustrates a perspective view of the implant shown in FIG. 14 with an outer covering removed according to an embodiment of the present disclosure.
FIG. 16 illustrates a perspective view of a frame of the implant shown in FIG. 14 in a compressed state according to an embodiment of the present disclosure.
FIG. 17 is a flowchart of an exemplary method according to an embodiment of the present disclosure.

### DETAILED DESCRIPTION

The following description and examples illustrate some example embodiments of the disclosure in detail. Those of skill in the art will recognize that there are numerous variations and modifications of the disclosure that are encompassed by its scope. Accordingly, the description of a certain example embodiment should not be deemed to limit the scope of the present disclosure.

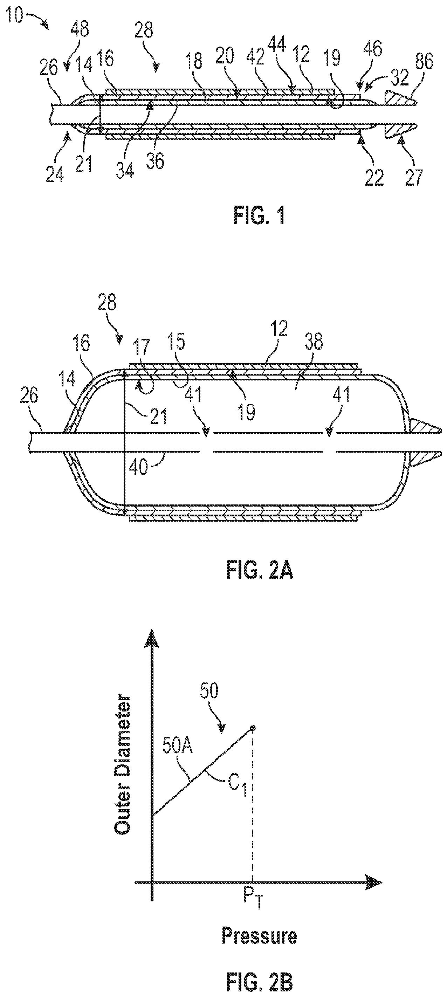

FIG. 1 illustrates a side cross sectional view of a system 10 for expansion of an expandable implant 12. The system may include an inflatable device 28 located on an elongate shaft 26. The elongate shaft 26 is connected to a nose cone 86 at a distal end 27 of the elongate shaft 26. The inflatable device 28 includes an interior inflatable body 14, which may be referred to as a first or inner inflatable body, and may include an exterior inflatable body 16, which may be referred to as a second or outer inflatable body. As shown in FIG. 1, the inflatable device 28, including the interior inflatable body 14 and the exterior inflatable body 16, is in a deflated state. The interior inflatable body 14 is configured to be inflated, and inflation of the interior inflatable body 14 causes inflation of the exterior inflatable body 16. The interior inflatable body 14 may be configured to inflate in response to receiving fluid. As will be described herein, the exterior inflatable body 16 may also be referred to as an exterior expandable body. While the exterior inflatable body 16 inflates when the interior inflatable body 14 inflates, in some embodiments, an isolated exterior inflatable body 16 may also inflate if fluid enters the exterior inflatable body 16 at a faster rate than the fluid exits the exterior inflatable body 16 via one or more openings.

The interior inflatable body 14 may include an outer wall 18 forming an outer surface 20 of the interior inflatable body 14. The interior inflatable body 14 may have a first (distal) portion 22 and a second (proximal) portion 24. The first (distal) portion 22 may have a first (distal) end and the second (proximal) portion 24 may have a second (proximal) end.

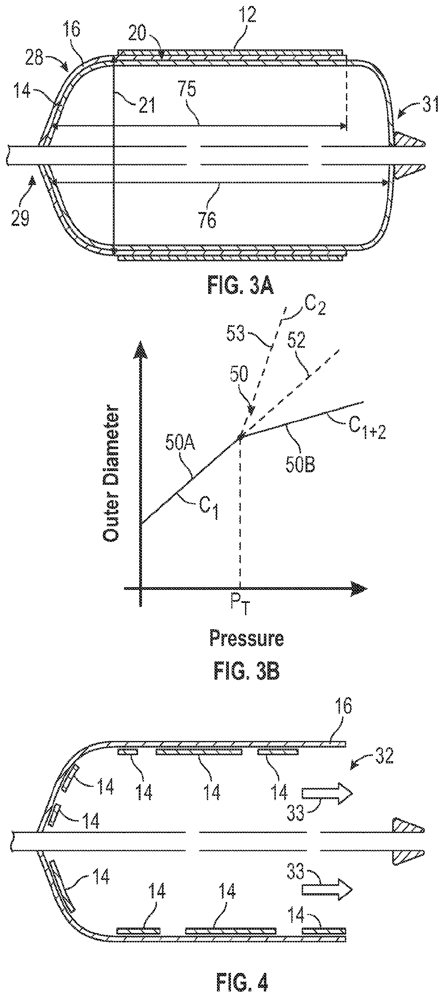

The first portion 22 and the second portion 24 may each be coupled to an elongate shaft 26 of a delivery apparatus configured to deliver the expandable implant 12 to a location in a patient's body. For example, the interior inflatable body 14 may be coupled to the elongate shaft 26 at the first (distal) portion 22 of the interior inflatable body 14 and may be coupled to the elongate shaft 26 at the second (proximal) portion 24 of the interior inflatable body 14. The interior inflatable body 14 may extend axially along a length of the elongate shaft 26 of the delivery apparatus in both a deflated state as shown in FIG. 1 and in an inflated state as shown in FIGS. 2A and 3A.

The interior inflatable body 14 may be configured to have a rounded profile when in an inflated state. The interior inflatable body 14 may extend radially outward from the elongate shaft 26 when in an inflated state and may extend around the axis of the elongate shaft 26. In some embodiments, the interior inflatable body 14 may have a tapered shape or may have another shape as desired.

The exterior inflatable body 16 may include an outer wall 42 forming an outer surface 44 of the exterior inflatable body 16. The exterior inflatable body 16 may have a first (distal) portion 46 and a second (proximal) portion 48. The first (distal) portion 46 may have a first (distal) end and the second (proximal) portion 48 may have a second (proximal) end. The exterior inflatable body 16 may have a diameter 21.

As shown, the exterior inflatable body 16 is not coupled to the elongate shaft 26 at the first (distal) portion 46 of the exterior inflatable body 16 and is coupled to the elongate shaft 26 at the second (proximal) portion 48 of the exterior inflatable body 16. However, in other embodiments, the exterior inflatable body 16 may be coupled to the elongate shaft 26 at the first (distal) portion 46 of the exterior inflatable body 16 and may also be coupled to the elongate shaft 26 at the second (proximal) portion 48 of the exterior inflatable body 16, or the exterior inflatable body 16 may be coupled to the elongate shaft 26 at the first (distal) portion 46 of the exterior inflatable body 16 and may not be coupled to the elongate shaft 26 at the second (proximal) portion 48 of the exterior inflatable body 16.

As illustrated, the exterior inflatable body 16 may have an opening 32 at the first (distal) portion 46 of the exterior inflatable body 16, as the exterior inflatable body 16 is disconnected to the elongate shaft 26 at the first (distal) portion 46 of the exterior inflatable body 16. In embodiments where the exterior inflatable body 16 is coupled to the elongate shaft 26 at the first (distal) portion 46 of the exterior inflatable body 16, there may be one or more openings instead of the opening 32, as will be described in further detail herein.

The exterior inflatable body 16 may have an inner wall 36 forming an inner surface 34. The inner surface 34 of the exterior inflatable body 16 contacts the outer surface 20 of the interior inflatable body 14. When the interior inflatable body 14 inflates, the exterior inflatable body 16 also inflates, as an expansion force from the interior inflatable body 14 is translated to the exterior inflatable body 16. The expandable implant 12 has an interior surface 19 that contacts the outer surface 44 of the exterior inflatable body 16. When the exterior inflatable body 16 inflates due to the inflation of the interior inflatable body 14, the exterior inflatable body 16 exerts an expansion force on the interior surface 19 of the expandable implant 12, causing the expandable implant 12 to dilate or expand.

The exterior inflatable body 16 may extend axially along the length of the elongate shaft 26 of the delivery apparatus in both a deflated state as shown in FIG. 1 and in an inflated state as shown in FIGS. 2A and 3A.

The exterior inflatable body 16 may be configured to have a generally rounded profile when in an inflated state. The exterior inflatable body 16 may extend radially outward from the elongate shaft 26 when in an inflated state and may extend around the axis of the elongate shaft 26. In some embodiments, the exterior inflatable body 16 may have a tapered shape or may have another shape as desired.

FIG. 2A illustrates a side-cross sectional view of the inflatable device 28 in a partially inflated state. The interior inflatable body 14 has an inner wall 15 forming an inner surface 17. The inner wall 15 of the interior inflatable body 14 may surround an interior chamber 38 of the interior inflatable body 14 that may be configured to hold fluid (for example, a liquid or other fluid in embodiments) for inflating the interior inflatable body 14. The interior chamber 38 may comprise a single chamber, as illustrated herein, or a plurality of chambers utilized to hold fluid for inflating the interior inflatable body 14. The interior chamber 38 may be sealed from outside of the interior inflatable body 14 by the coupling of the proximal end and the distal end of the interior inflatable body 14 to the elongate shaft 26.

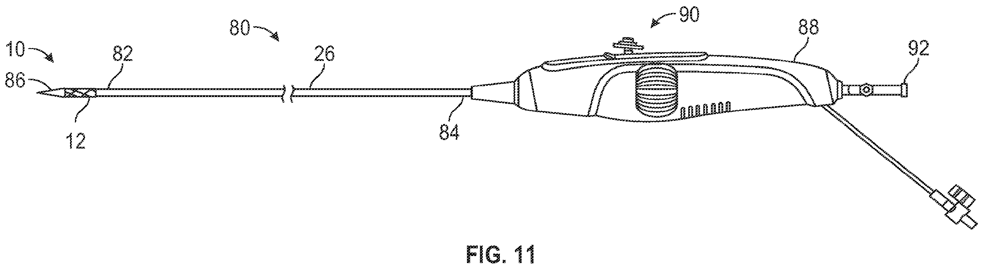

An inflation lumen 40 may be provided that is configured to inflate the inflatable device 28. More specifically, the inflation lumen 40 may be configured to inflate the interior inflatable body 14 of the inflatable device 28. The inflation lumen 40 may extend within the interior chamber 38 of the interior inflatable body 14 and may include channels 41 configured for fluid to be passed into, in order to fill the interior chamber 38 and thus inflate the interior inflatable body 14. Further, the inflation lumen 40 may be configured to withdraw fluid from the interior chamber 38 through the channels 41 to deflate the interior inflatable body 14. The inflation lumen 40 may be in fluid communication with a port 92 (as shown in FIG. 11) that may be utilized to pass fluid into or out of the inflation lumen 40 to control the inflation of the inflatable device 28. The inflation lumen 40 may be one or more inflation lumens. The inflation lumen 40 may extend along the elongate shaft 26 and may be configured to inflate one or more of the interior inflatable body 14 or the exterior inflatable body 16.

FIG. 2B illustrates a curve 50 representing compliance (or expansion rate) of the inflatable device 28. The interior inflatable body 14 and the exterior inflatable body 16 each have a compliance, which refers to the ability to expand when a force, such as an expansion force, is exerted onto the interior inflatable body 14 or the exterior inflatable body 16. In particular, the interior inflatable body 14 has a first compliance C₁ and the exterior inflatable body 16 has a second compliance C₂. The interior inflatable body 14 may expand at a faster rate than the exterior inflatable body 16 when the same amount of pressure is applied to both the interior inflatable body 14 and the exterior inflatable body 16. Thus, the first compliance C₁ of the interior inflatable body 14 is greater than the second compliance C₂ of the exterior inflatable body 16.

As shown in FIG. 2B, according to the curve 50, for which a first portion 50A is shown, the outer diameter of the inflatable device 28 increases as pressure is increased. The pressure increase is caused by the fluid introduced into the interior chamber 38 of the interior inflatable body 14 by the inflation lumen 40. The exterior inflatable body 16 may have a larger diameter than the interior inflatable body 14 when in a deflated state. Thus, while the interior inflatable body 14 is being inflated and experiencing hoop stress (or expanding beyond a resting state), the exterior inflatable body 16 may not initially experience hoop stress. Accordingly, the compliance of the inflatable device 28 is equal to the compliance of the interior inflatable body 14, the first compliance C₁ until a threshold pressure, P_{T}, is reached. The threshold pressure, P_{T}, is reached when the exterior inflatable body 16 begins to experience hoop stress. The exterior inflatable body 16 may experience hoop stress when the outer diameter of the interior inflatable body 14 is at least as large as the inner diameter of the exterior inflatable body. The threshold pressure P_{T} can also be represented by an amount of fluid received by the interior inflatable body 14 that causes the threshold pressure P_{T}.

FIG. 3A illustrates a side-cross sectional view of the inflatable device 28 in a fully inflated state. The interior inflatable body 14 is fully inflated and the exterior inflatable body 16 (or "exterior expandable body") is also fully inflated. The expandable implant 12 is fully expanded and capable of being installed. The interior inflatable body 14 has a length 76 and the exterior inflatable body 16 has a length 75 that is less than the length 76. In some embodiments, the length 75 is sufficiently long such that the expandable implant 12 contacts the exterior inflatable body 16, but the expandable implant 12 does not directly contact the interior inflatable body 14. As the interior inflatable body 14 inflates, causing the exterior inflatable body 16 to also inflate, the portion of the outer surface 20 of the first inflatable body 14 that is covered by the exterior inflatable body 16 may decrease. That is, as the interior inflatable body 14 and the exterior inflatable body 16 inflate, the exterior inflatable body 16 may peel back from the interior inflatable body 14 in a direction toward the proximal end 29 (opposite the distal end 31) of the inflatable device 28. Accordingly, the ratio of the length 75 to the length 76 may decrease as the interior inflatable body 14 and the exterior inflatable body 16 inflate.

FIG. 3B illustrates the curve 50, for which the first portion 50A and a second portion 50B are shown, representing compliance of the inflatable device 28. During the second portion 50B where pressure in the inflatable device 28 exceeds the threshold pressure, P_{T}, the interior inflatable body 14 may be inflated and experience hoop stress. In addition, the exterior inflatable body 16 may also be inflated and experience hoop stress (and expand beyond the resting state). However, because the exterior inflatable body 16 has a lower compliance (C₂), the expansion of the interior inflatable body 14 is limited by the exterior inflatable body 16. Thus, the compliance of the inflatable device 28 in the second portion 50B is based on the first compliance C₁ as well as the second compliance C₂. Comparatively, the hoop stress experienced by the exterior inflatable body 16 is less than the hoop stress experienced by the interior inflatable body 14. In addition, the exterior inflatable body 16 experiences very little axial stress, significantly reducing risk of radial failure of the exterior inflatable body 16.

Also shown is a projected curve 52 of the compliance of the interior inflatable body 14. If not for the presence of the exterior inflatable body 16, the outer diameter of the inflatable device 28 would continue to increase with pressure according to the first compliance C₁ of the interior inflatable body 14. Thus, the curve 50 is based on the compliance of the interior inflatable body 14 when the inflation pressure is below the threshold pressure, and the curve 50 is based on the combined compliance of the exterior inflatable body 16 and the compliance of the interior inflatable body 14 when the inflation pressure is above the threshold pressure. The inflatable device has a first expansion rate when the interior inflatable body is below a threshold inflation pressure and the inflatable device has a second expansion rate when the interior inflatable body is above the threshold pressure, the second expansion rate being lower than the first expansion rate. The dual-compliance (or variable compliance) nature of the inflatable device 28 may allow for the interior inflatable body 14 to be inflated at a faster rate than conventional balloons, as the exterior inflatable body 16 will restrict the expansion of the interior inflatable body 14, reducing the risk of the interior inflatable body 14 bursting. Also shown is projected curve 53, which represents the compliance C₂ of the exterior inflatable body 16.

As shown in FIGS. 1, 2A, and 3A, the exterior inflatable body 16 (or "exterior expandable body") at least partially surrounding the interior inflatable body 14 shields the interior inflatable body 14 from puncture. In conventional systems, a balloon may be vulnerable to puncture by a stray wire or other component. In addition, the exterior inflatable body 16 provides pressure support to the interior inflatable body 14 as the interior inflatable body 14 is being inflated, to provide stability to the inflatable device 28.

While FIGS. 1, 2A, and 3A illustrate the inflatable device 28 exerting an expansion force onto an interior surface 19 of the expandable implant 12, the systems and methods described herein may be used to exert an expansion force onto any surface, such as a passage or space within a patient's body. For example, the systems and methods described herein may be used to dilate a blood vessel of the patient.

Upon the expandable implant 12 being fully deployed, the interior inflatable body 14 and the exterior inflatable body 16 may be deflated in a reverse sequence than shown in FIGS. 1, 2A, and 3A. The interior inflatable body 14, the exterior inflatable body 16, and the elongate shaft 26 may be removed from the implantation site, with the deployed expandable implant 12 remaining in position.

FIG. 4 illustrates the interior inflatable body 14 being in a punctured or a burst state. As shown, the interior inflatable body 14 may be separated into a plurality of pieces (or portions or fragments). The exterior inflatable body 16 may surround and capture the interior inflatable body 14 so that a piece or fragment of the interior inflatable body 14 does not become located within a patient in an uncontrolled manner. In conventional balloon systems, when the balloon bursts, fragments of the burst balloon may be difficult to retrieve, as the fragments may move in an unpredictable and uncontrollable manner.

In addition, when the interior inflatable body 14 is punctured or bursts, the fluid located within the interior chamber 38 of the interior inflatable body 14 may exit the interior inflatable body 14. The presence of the exterior inflatable body 16 allows for a controlled release of the fluid to the surrounding environment. The opening 32 of the exterior inflatable body 16 directs the fluid in a distal direction 33 and also ensures that the exterior inflatable body 16 does not also burst. When the direction in which the fluid is released can be predicted, additional safeguards and contingency measures may be planned, in case the interior inflatable body 14 is punctured or bursts. In conventional balloon systems, when the balloon bursts, the fluid may exit the balloon in an unpredictable and uncontrollable manner.

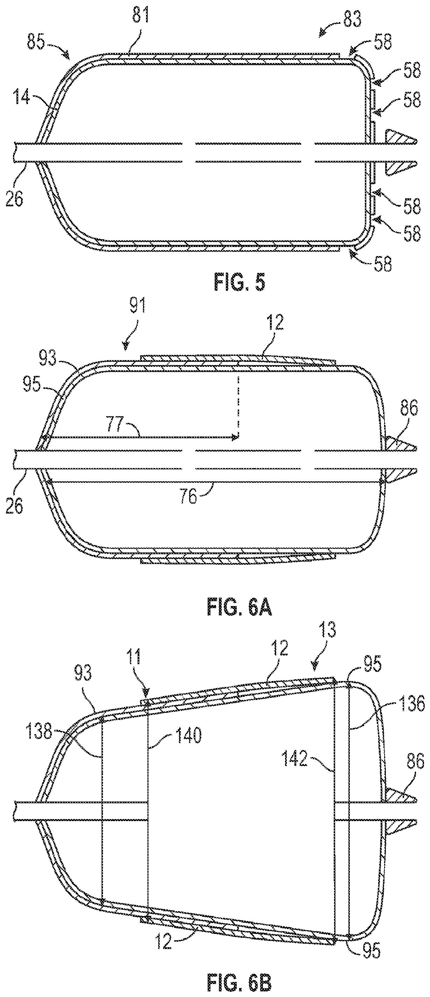

FIG. 5 illustrates an embodiment where an exterior inflatable body 81 (or "exterior expandable body") has one or more openings 58 instead of a single opening 32, shown in FIG. 4. The one or more openings 58 may be located on a first (distal) portion 83 of the exterior inflatable body 16. The exterior inflatable body 81 may be coupled to the elongate shaft 26 at the first (distal) portion 83 and the second (proximal) portion 85. In this way, the exterior inflatable body 81 more completely covers the interior inflatable body 14 than when a single opening 32 (shown in FIG. 4) is used, providing increased puncture shielding and retention of one or more pieces or fragments of the interior inflatable body 14 if the interior inflatable body 14 bursts.

The one or more openings 58 allow for a controlled release of the fluid within the interior inflatable body 14 in the event of a puncture or bursting of the interior inflatable body 14, similar to the inflatable device 28 shown in FIG. 4. The number and location of the one or more openings 58 may depend on the dimensions of the inflatable device as well as the specific application of the inflatable device.

FIG. 6A illustrates an inflatable device 91 with an exterior inflatable body 93 (or "exterior expandable body") having a length 77 that is approximately half the length 76 of the interior inflatable body 95. In this embodiment, the expandable implant 12 may be partially located on the outer surface of the exterior inflatable body 93 and partially located on the outer surface of the interior inflatable body 95.

FIG. 6B illustrates the inflatable device 91 of FIG. 6A in a further inflated state. As the exterior inflatable body 93 has a length 77 that is only a portion of the length 76 of the interior inflatable body 95, when the interior inflatable body 95 is further inflated, a diameter 136 at a distal portion of the interior inflatable body 95 may be greater than a diameter 138 at a proximal portion of the interior inflatable body 95. The proximal portion of the interior inflatable body 95 may have a smaller diameter 138 because of the exterior inflatable body 93 being located around the proximal portion of the interior inflatable body 95 and having a lower compliance than the interior inflatable body 95.

As a result, the expandable implant 12 may be angled such that a diameter 142 of the expandable implant 12 at a distal end 13 of the expandable implant 12 is greater than a diameter 140 at a proximal end 11 of the expandable implant 12. Many aspects of the inflatable device 91 may be tuned to adjust the shape of the inflatable device 91 when inflated, such as a thickness of the interior inflatable body 95, a thickness of the exterior inflatable body 93, a material the interior inflatable body 95 is made of, a material the exterior inflatable body 93 is made of, or an amount of fluid used to inflate the interior inflatable body 95.

The tapered profile of the inflatable device 91, which may be referred to as being "A" shaped, may be advantageous, as the expandable implant 12 may not move in a distal direction (toward the nose cone 86) and slip off of the inflatable device 91 due to the shape of the inflatable device 91 shown in FIG. 6B. If the expandable implant 12 slips off of the inflatable device 91, it may be very difficult to retrieve the expandable implant 12 from within the patient's body. The expandable implant 12 may have the interior surface 19 of the expandable implant 12 being wider at a distal end 13 of the expandable implant than at a proximal end 11 of the expandable implant. In embodiments, the expandable implant 12 may comprise an "A" shaped or "V" shaped implant having a frame with one end being wider than another end. For example, an "A" shaped implant may have the distal end being wider than a proximal end, and a "V" shaped implant may have the proximal end being wider than the distal end. Various forms of implants with angled frames may be utilized as desired.

While a gap may be shown between the expandable implant 12 and the outer surface of the interior inflatable body 95, in many embodiments, the thickness of the exterior inflatable body 93 is a thinness that renders the illustrated gap to be negligible and not affecting the performance of the systems described herein.

In addition, while an inflatable device 91 having an "A" shaped profile is shown in FIGS. 6A-6B, in some embodiments, the exterior inflatable body 93 may be attached to the elongate shaft 26 at a distal portion of the exterior inflatable body 93 and not attached to the elongate shaft 26 at a proximal portion of the exterior inflatable body 93. Thus, in these embodiments, a diameter at the proximal portion of the inflatable device 91 is greater than a diameter at a distal portion of the inflatable device 91, forming a "V" shaped profile. A "V" shaped profile may be advantageous in some situations, such as when the expandable implant 12 has a "V" shaped profile as well.

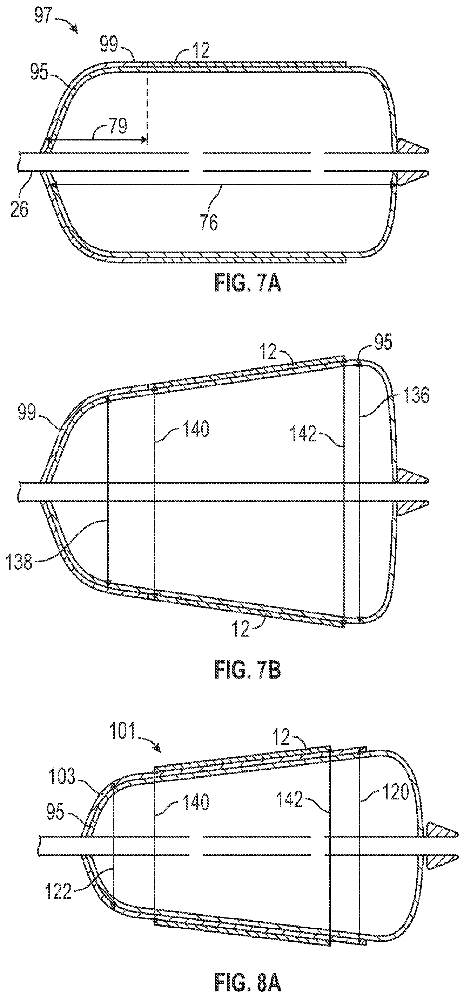

FIG. 7A illustrates an inflatable device 97 with an exterior inflatable body 99 (or "exterior expandable body") having a length 79 that is approximately a quarter of the length 76 of the interior inflatable body 95. In this embodiment, the expandable implant 12 may not be located on the outer surface of the exterior inflatable body 99 and located on the outer surface of the interior inflatable body 95.

FIG. 7B illustrates the inflatable device 97 of FIG. 7A in a further inflated state. As the exterior inflatable body 99 has a length 79 that is only a portion of the length 76 of the interior inflatable body 95, when the interior inflatable body 95 is further inflated, a diameter 136 at a distal portion of the interior inflatable body 95 may be greater than a diameter 138 at a proximal portion of the interior inflatable body 95. The proximal portion of the interior inflatable body 95 may have a smaller diameter 138 because of the exterior inflatable body 99 being located around the proximal portion of the interior inflatable body 95 and having a lower compliance than the interior inflatable body 95.

As a result, the expandable implant 12 may be angled such that a diameter 142 of the expandable implant 12 at a distal end 13 of the expandable implant 12 is greater than a diameter 140 at a proximal end 11 of the expandable implant 12. Many aspects of the inflatable device 97 may be tuned to adjust the shape of the inflatable device 97 when inflated, such as a thickness of the interior inflatable body 95, a thickness of the exterior inflatable body 99, a material the interior inflatable body 95 is made of, a material the exterior inflatable body 99 is made of, or an amount of fluid used to inflate the interior inflatable body 95.

As described herein, the tapered profile of the inflatable device 97, which may be referred to as being "A" shaped, may be advantageous, as the expandable implant 12 may not move in a distal direction (toward the nose cone 86) and slip off of the inflatable device 97 due to the shape of the inflatable device 97 shown in FIG. 7B. If the expandable implant 12 slips off of the inflatable device 97, it may be very difficult to retrieve the expandable implant 12 from within the patient's body.

While an inflatable device 97 having an "A" shaped profile is shown in FIGS. 7A-7B, in some embodiments, the exterior inflatable body 99 may be attached to the elongate shaft 26 at a distal portion of the exterior inflatable body 99 and not attached to the elongate shaft 26 at a proximal portion of the exterior inflatable body 99. Thus, in these embodiments, a diameter at the proximal portion of the inflatable device 97 is greater than a diameter at a distal portion of the inflatable device 97, forming a "V" shaped profile. A "V" shaped profile may be advantageous in some situations, such as when the expandable implant 12 has a "V" shaped profile as well.

FIG. 8A illustrates an inflatable device 101 having a tapered "A" shaped profile. That is, the diameter 120 at a distal portion of the inflatable device 101 (or distal portion of the exterior inflatable body 103 or distal portion of the interior inflatable body 95) is greater than the diameter 122 at a proximal portion (or proximal portion of the exterior inflatable body 103 or proximal portion of the interior inflatable body 95) of the inflatable device 101. The "A" shaped profile results in the diameter 142 of the expandable implant 12 at a distal end of the expandable implant 12 being greater than a diameter 140 at a proximal end of the expandable implant 12.

The inflatable device 101 may have the "A" shaped profile while having the exterior inflatable body 103 cover a substantial (e.g., greater than 60%, greater than 70%, greater than 75%, greater than 80%, greater than 90%) of the interior inflatable body 95. The inflatable devices shown in FIGS. 6A-6B and 7A-7B achieve the "A" shaped profile using exterior inflatable bodies that cover a much smaller portion of the interior inflatable body. However, the inflatable device 101 shown in FIG. 8A is able to shield a greater surface area of the interior inflatable body 95 while also achieving the "A" shaped profile.

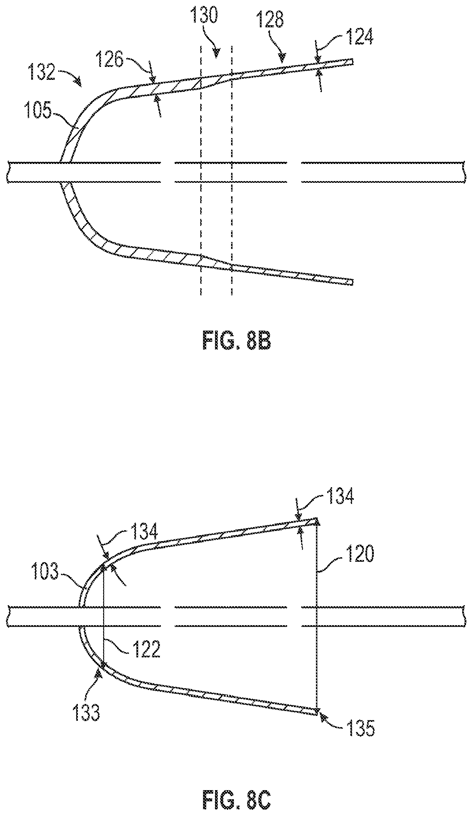

FIG. 8B illustrates a cross-sectional view of an exterior inflatable body 105 (or "exterior expandable body") isolated from the rest of an inflatable device. The exterior inflatable body 105 may be separated into three sections - a proximal section 132, a middle section 130, and a distal section 128. The thickness 126 of the exterior inflatable body 105 in the proximal section 132 is greater than the thickness 124 of the exterior inflatable body 105 in the distal section 128. The thickness of the middle section 130 may transition from the thickness 126 of the exterior inflatable body 105 in the proximal section 132 to the thickness 124 of the exterior inflatable body 105 in the distal section 128. The thickness may be greater at a proximal portion of the exterior inflatable body 105 than at the distal portion of the exterior inflatable body 105. While three sections are illustrated, other embodiments with any number of sections may be possible. For example, there may be two sections, with a first section having a uniform thickness and a second section with a thickness that tapers to be thinner as the exterior inflatable body 105 extends from the proximal end to the distal end. In another example, there may be five sections with three sections having respective uniform thicknesses separated by two transitional sections of tapering thickness. In yet another example, there may only be one section with a thickness that tapers to be thinner as the exterior inflatable body 105 extends from the proximal end to the distal end.

FIG. 8C illustrates a cross-sectional view of the exterior inflatable body 103 of the embodiment of FIG. 8A isolated from the rest of the inflatable device 101. Unlike the exterior inflatable body 105 of FIG. 8B, the exterior inflatable body 103 has a uniform thickness 134. However, the exterior inflatable body 105 of FIG. 8C has a shape that widens, from the proximal end 133 of the exterior inflatable body 103 to the distal end 135 of the exterior inflatable body 103. That is, a diameter 122 at a proximal portion of the exterior inflatable body 103 is smaller than a diameter 120 at a distal portion of the exterior inflatable body 103. This shape provides the "A" shaped profile for the inflatable device 101 shown in FIG. 8A. The shapes of the inflatable devices shown in FIGS. 6B, 7B, and 8A may be a conical frustum shape.

While an inflatable device 101 having an "A" shaped profile may be shown in FIGS. 8A-8C, in some embodiments, the exterior inflatable body may be attached to the elongate shaft 26 at a distal portion of the exterior inflatable body and not attached to the elongate shaft 26 at a proximal portion of the exterior inflatable body. Thus, in these embodiments, a diameter at the proximal portion of the inflatable device 101 is greater than a diameter at a distal portion of the inflatable device 101, forming a "V" shaped profile. A "V" shaped profile may be advantageous in some situations, such as when the expandable implant 12 has a "V" shaped profile as well.

In some embodiments, as the inflation pressure increases and the exterior inflatable body experiences hoop stress, the exterior inflatable body may expand at a faster rate than the interior inflatable body.

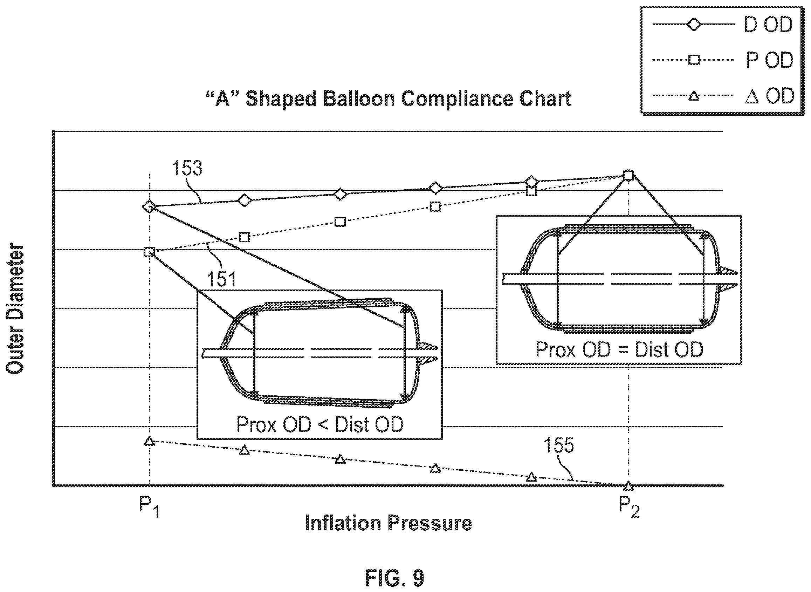

FIG. 9 illustrates a graph showing outer diameter of an inflatable device based on inflation pressure, with a first curve 151 representing the compliance of the inflatable device at a proximal portion of the inflatable device and a second curve 153 representing the compliance of the inflatable device at a distal portion of the inflatable device. A third curve 155 shows the difference between the second curve 153 and first curve 151. As described herein, the inflatable device has both the exterior inflatable body and the interior inflatable body located at the proximal portion of the inflatable device and the interior inflatable body located at the distal portion of the inflatable device.

At lower inflation pressures, such as P₁, the exterior inflatable body (or "exterior expandable body") may significantly restrict the outward expansion of the interior inflatable body at the proximal portion of the inflatable device, causing a difference between the outer diameter of the inflatable device at the proximal portion (shown in first curve 151) and the outer diameter of the inflatable device at the distal portion (shown in second curve 153).

As the inflation pressure increases, the exterior inflatable body may begin to expand at a faster rate, and the first curve 151 and the second curve 153 may converge at P₂. When the first curve 151 and the second curve 153 converge, the outer diameter of the inflatable device at the proximal portion and the outer diameter of the inflatable device at the distal portion may be the same, resulting in an "H" shaped profile of the inflatable device.

In embodiments, portions of the interior inflatable body and exterior inflatable body may be covered with materials. For example, coatings or other coverings may be positioned over the inflatable bodies. A coating may cover the outer surface of the exterior inflatable body, yet the outer surface may apply an expansion force to the expandable implant through the coating. Combinations of features across various embodiments and other variations may be utilized as desired.

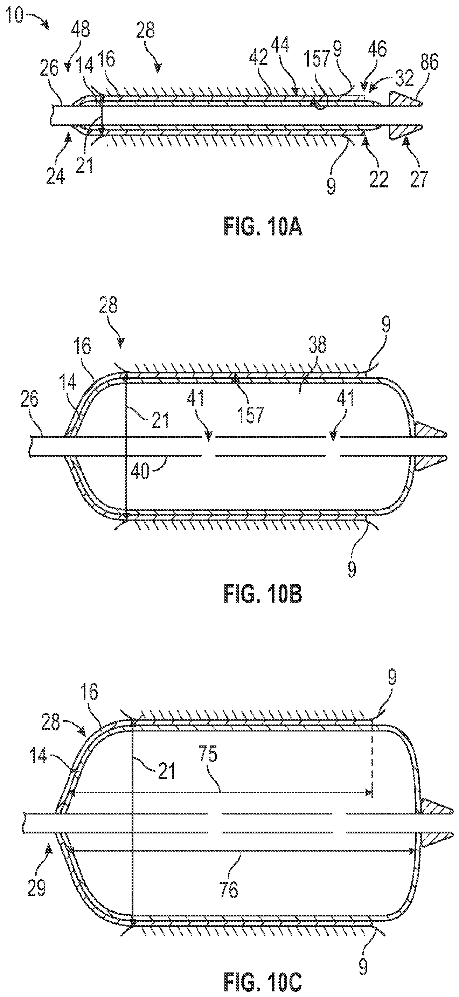

As noted herein, while an expandable implant 12 has been illustrated, the systems disclosed herein may be used to provide an expansion force onto any surface within the patient's body to dilate a passage or space within the patient. FIGS. 10A-10C illustrate the system 10 being used to provide an expansion force onto an interior surface 157 of a passage or space 9 within a patient's body. The passage or space 9 may be a blood vessel, urethra, or any other body within the patient. All other components of embodiments may remain the same as described herein.

The system may be utilized as part of a delivery system for the expandable implant. FIG. 11, for example, illustrates a delivery apparatus 80 that may be utilized to deliver the expandable implant 12 to a location in a patient's body. The delivery apparatus 80 may include the elongate shaft 26, which may have a distal portion 82 and a proximal portion 84. The system 10 including the inflatable bodies 14, 16 may be positioned on the distal portion 82 of the elongate shaft 26. The elongate shaft 26 may include a nose cone 86. The elongate shaft 26 may comprise a guide wire lumen for a guide wire to extend through as the delivery apparatus 80 approaches an implantation site. The nose cone 86 may be positioned distal of the inflatable bodies 14, 16. In embodiments, the inflatable bodies 14, 16 may be positioned adjacent to the nose cone 86.

The proximal portion 84 of the elongate shaft 26 may be coupled to a housing in the form of a handle 88. The handle 88 may be configured to be gripped by a user to control movement of the elongate shaft 26. The delivery apparatus 80 may include an actuation mechanism 90 for actuating operation of the delivery apparatus 80, which may include deflecting the elongate shaft 26 into a desired orientation. For example, the elongate shaft 26 may be configured to be flexible to deflect to the desired portion of the patient's body, and may be steerable with operation of the actuation mechanism 90.

A proximal end of the delivery apparatus 80 may include a port 92 for passing fluid into and out of the inflation lumen 40.

The configuration of the delivery apparatus may vary from the configuration shown in FIG. 11. Other types of delivery apparatuses may be utilized than shown in FIG. 11.

The implant 12 may be a mitral, tricuspid, or pulmonary prosthetic valve, among other forms of prosthetic heart valves or prosthetics. The implant 12 may comprise a stent, clip, or other form of implant that may be inserted in a portion of the patient's body, including the patient's heart.

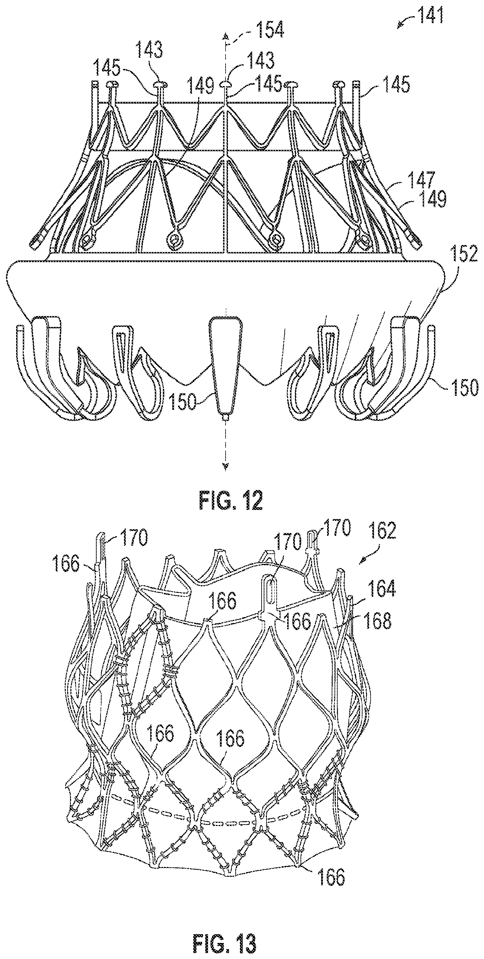

FIG. 12, for example, illustrates an implant 141 including couplers 143 in the form of tabs at a proximal end of the implant 141. The couplers 143 may be positioned at the ends of struts 145 of a frame 147 of the implant 141. The implant 141 may include proximal anchors 149 and may include distal anchors 150. The anchors may be configured to secure the implant to a native valve location. The implant 141 may comprise a prosthetic replacement mitral heart valve, and the distal anchors 150 may extend over leaflets of the native mitral valve. The proximal anchors 149 may be positioned on the atrial side of the native mitral valve. The implant 141 may include a skirt 152 and may extend around an axis 154.

The implant 141 may comprise a self-expanding implant, and may be configured to expand within a patient's body upon being released from an implant retention area of a delivery apparatus. For example, the delivery apparatus may have a capsule covering the implant and then the capsule may be retracted from the implant to uncover the self-expanding implant and allow the implant to expand. Such an implant may be made of a nitinol material (e.g., a nitinol frame) or other shape memory material as desired. FIG. 12 illustrates the implant 141 in an expanded or deployed state.

Other forms of implants may include prosthetic replacement aortic valves. FIG. 13, for example, illustrates an embodiment of a prosthetic replacement aortic valve. The implant 162 may be an expandable implant as shown in FIG. 13, which may be configured to be expanded to be placed in position within the native valve location. The implant 162 may include a frame 164 including a plurality of supports 166 configured to be compressed for positioning within the delivery apparatus and configured to be expanded at the desired time. The frame 164 may support prosthetic valve leaflets 168 that operate in lieu of the native valve leaflets. The frame 164 may include couplers 170 for coupling to the delivery apparatus, to retain the implant to the delivery apparatus until deployment is desired. The couplers 170 may comprise apertures as shown in FIG. 13, or may have other forms as desired.

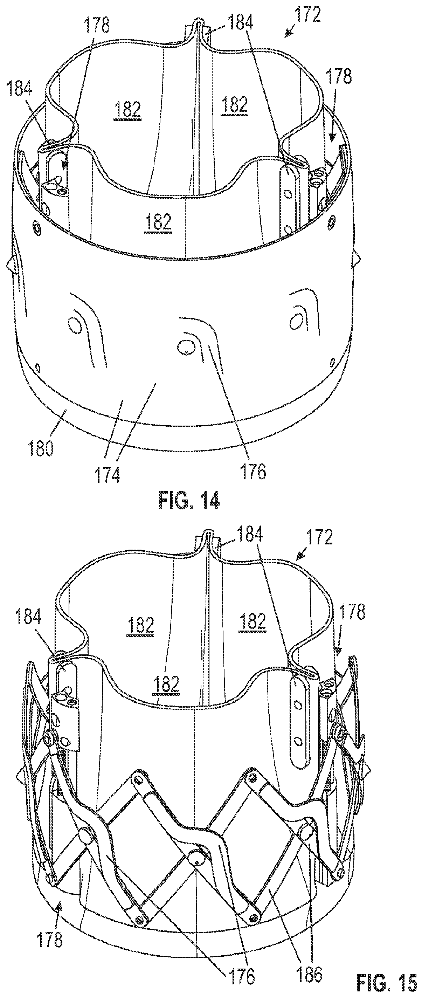

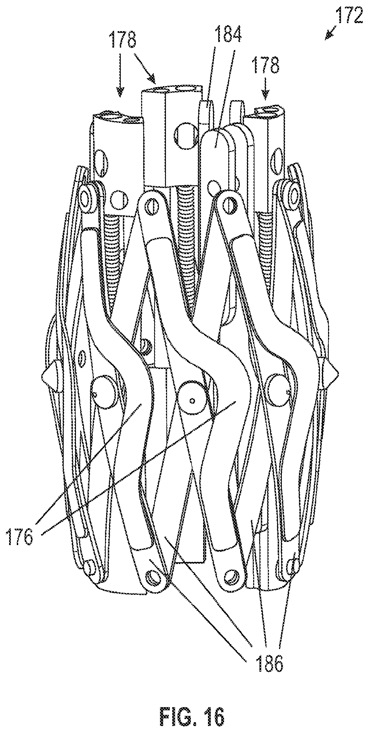

Other forms of implants may include mechanically expandable implants. A mechanically expandable implant may expand due to operation of a mechanical assembly. An example of such an implant is disclosed in U.S. Patent No. 9,913,716, filed January 24, 2017 and issued March 13, 2018. FIGS. 72, 77, and 81 of U.S. Patent No. 9,913,716 are reproduced here as FIGS. 14-16. The implant may include a prosthetic replacement heart valve assembly 172, a stent lattice 174, graft enclosures 176, jack assemblies 178, graft material 180, valve leaflets 182, and commissure plates 184. A cover is removed in FIG. 15 to show struts 186. FIG. 16 illustrates the implant with the cover removed, and in a compressed state. A crimping device may be utilized to move the implant to a compressed state as shown in FIG. 16. A mechanical assembly may then be utilized to expand the implant at a desired location within the patient's body.

Other forms of implants such as stents or filters, among others, may be configured similarly as the implants disclosed herein. For example, the implants utilized according to embodiments herein may have an angled interior profile as discussed herein, or may have other profiles as desired. The implants may be cylindrical and may have a uniform interior profile in embodiments, for example. The implants may be configured to expand radially outward from an axis that the implant surrounds, for example a longitudinal axis of the implant.

The delivery apparatus and apparatuses and the systems disclosed herein may be used in a variety of procedures, which may include transcatheter aortic valve implantation (TAVI). The delivery apparatus and the systems disclosed herein may be utilized for transarterial access, including transfemoral access, to a patient's heart. In embodiments, the delivery apparatus may be utilized for mitral, tricuspid, and pulmonary replacement and repair as well. The delivery systems may be utilized in transcatheter percutaneous procedures, including transarterial procedures, which may be transfemoral or transjugular. Transapical procedures, among others, may also be utilized.

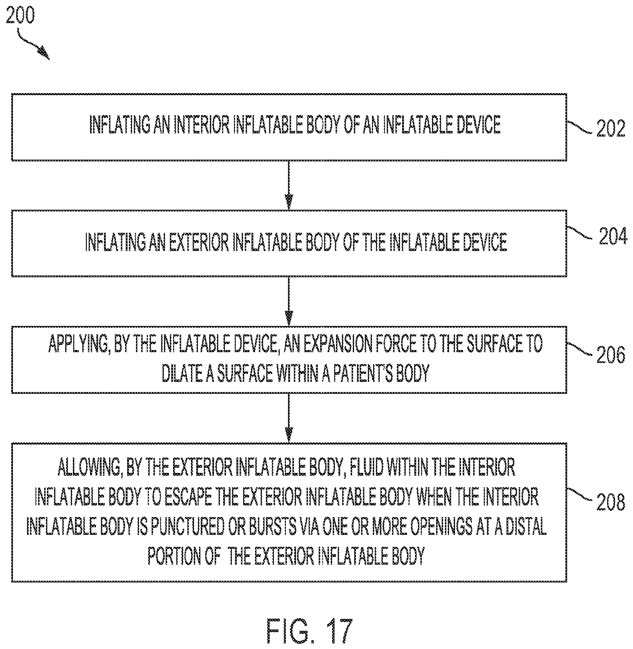

FIG. 17 illustrates a flowchart of a process 200 for dilation of a surface within a patient's body. An interior inflatable body (e.g., interior inflatable body 14) of an inflatable device (e.g., inflatable device 28) is inflated (step 202). The interior inflatable body may be inflated by an inflation lumen (e.g., inflation lumen 40) of an elongate shaft (e.g., elongate shaft 26) that the inflatable device is coupled to. The interior inflatable body may be inflated using a fluid.

An exterior inflatable body (e.g., exterior inflatable body 16) is inflated (step 204). The exterior inflatable body at least partially surrounds the interior inflatable body and the exterior inflatable body inflates when the interior inflatable body inflates. The exterior inflatable body may have a lower compliance than the interior inflatable body, thereby limiting expansion of the interior inflatable body when the interior inflatable body is inflated.

As the interior inflatable body and the exterior inflatable body inflate, the inflatable device applies an expansion force to a surface to dilate the surface within the patient's body (step 206). As described herein, the surface may be an interior surface of an expandable implant or may be a passage or space within the patient's body.

The exterior inflatable body may shield the interior inflatable body from puncture and may also provide pressure support during inflation, as described herein.

As an optional possible step, in the event of puncture, the exterior inflatable body may retain one or more portions of the interior inflatable body when the interior inflatable body is punctured or bursts. In addition, one or more openings (e.g., opening 32 or one or more openings 58) may allow the fluid within the interior inflatable body to escape the exterior inflatable body in a controlled and directed manner when the inflatable body is punctured or bursts (step 208).

Steps of methods disclosed herein may be modified as desired, including adding, removing, or substituting steps as desired. Methods as disclosed herein may be utilized in locations that do not utilize native valves, including a pulmonary artery and in the vena cava, among other locations (other arteries, blood vessels, or other vasculature of a patient's body, among other portions of a patient's body). An implant such as a stent or other form of implant may be delivered to such portions of the patient's body.

The user as disclosed herein may comprise a surgeon, physician, or other medical professional, among other users.

Features of embodiments may be modified, substituted, excluded, or combined.

In addition, the methods herein are not limited to the methods specifically described, and may include methods of utilizing the systems and apparatuses disclosed herein.

The steps of the method may be modified, excluded, or added to, with systems, apparatuses, and methods disclosed herein.

The features of the embodiments disclosed herein may be implemented independently of the delivery apparatuses, or independent of other components disclosed herein. The various apparatuses of the systems may be implemented independently.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein. Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

Certain embodiments of systems, apparatuses, and methods are described herein, including the best mode known to the inventors for carrying out the same. Of course, variations on these described embodiments will become apparent to those of ordinary skill in the art upon reading the foregoing description. The inventor expects skilled artisans to employ such variations as appropriate, and the inventors intend for the systems, apparatuses, and methods to be practiced otherwise than specifically described herein. Moreover, any combination of the above-described embodiments in all possible variations thereof is encompassed by the systems, apparatuses, and methods unless otherwise indicated herein or otherwise clearly contradicted by context.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses an approximation that may vary, yet is capable of performing the desired operation or process discussed herein.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed.

## Claims

1. A system (10) for dilation within a patient's body of a surface, the system comprising:
an inflatable device (28; 91; 97; 101) having:
an interior inflatable body (14; 95) configured to inflate in response to receiving fluid; and
an exterior inflatable body (16; 81; 93; 99; 103; 105) at least partially surrounding the interior inflatable body (14; 95) and configured to inflate when the interior inflatable body (14; 95) inflates and apply an expansion force to the surface to dilate the surface, the exterior inflatable body (16; 81; 93; 99; 103; 105) having a distal portion (46), a proximal portion (48), and one or more openings (32; 58) at the distal portion (46) configured to allow the fluid within the interior inflatable body to escape the exterior inflatable body (16; 81; 93; 99; 103; 105) when the interior inflatable body (14; 95) is punctured or bursts,
wherein the inflatable device (28; 91; 97; 101) has a first expansion rate when the interior inflatable body (14; 95) is below a threshold inflation pressure and the inflatable device has a second expansion rate when the interior inflatable body (14; 95) is above the threshold inflation pressure, the second expansion rate being lower than the first expansion rate.

2. The system (10) of claim 1, wherein the exterior inflatable body (16; 81; 93; 99; 103; 105) has lower compliance (C2) than the interior inflatable body (14; 95) to limit expansion of the interior inflatable body (14; 95).

3. The system (10) of claim 1 or 2, wherein the exterior inflatable body (16; 81; 93; 99; 103; 105) expands beyond a resting state when the interior inflatable body (14; 95) is inflated to the threshold inflation pressure.

4. The system of any of claims 1 to 3, wherein the first expansion rate is based on compliance (C1) of the interior inflatable body (14; 95) and the second expansion rate is based on compliance (C2) of the exterior inflatable body (16; 81; 93; 99; 103; 105) and the compliance (c2) of the interior inflatable body (14; 95).

5. The system (10) of any of claims 1 to 4, wherein the exterior inflatable body (16; 81; 93; 99; 103; 105) shields the interior inflatable body (14; 95) from puncture, or wherein the exterior inflatable body (16; 81; 93; 99; 103; 105)is configured to provide pressure support during inflation of the interior inflatable body (14; 95).

6. The system (10) of any of claims 1 to 5, wherein the exterior inflatable body (16; 81; 93; 99; 103; 105) is configured to retain one or more portions of the interior inflatable body (14; 95) when the interior inflatable body (14; 95) is punctured or bursts.

7. The system (10) of any of claims 1 to 6, wherein the exterior inflatable body (16; 81; 93; 99; 103; 105) is coupled to a shaft (26) of a delivery apparatus (80) at the proximal portion (48) of the exterior inflatable body (16; 81; 93; 99; 103; 105) and the exterior inflatable body (16; 81; 93; 99; 103; 105) is disconnected to the shaft of the delivery apparatus at the distal portion (46) of the exterior inflatable body (16; 81; 93; 99; 103; 105), or is coupled to the shaft (26) of the delivery apparatus (80) at the distal portion (46) of the exterior inflatable body (16; 81; 93; 99; 103; 105).

8. The system (10) of any of claims 1 to 7, wherein the interior inflatable body (14; 95) has a first length (76) and the exterior inflatable body (16; 81; 93; 99; 103; 105) has a second length (75) that is less than the first length (76).

9. The system (10) of any of claims 1 to 8, wherein the surface comprises an interior surface (19) of an expandable implant (12; 141; 162).

10. The system (10) of any of claims 1 to 9, wherein a diameter (136) of the interior inflatable body (95) at a distal portion (46) of the interior inflatable body (14; 95) is greater than a diameter (138) of the exterior inflatable body (16; 81; 93; 99; 103; 105) at the proximal portion (46) of the exterior inflatable body (16; 81; 93; 99; 103; 105), and wherein the surface comprises an interior surface (19) of an expandable implant being wider at a distal end of the expandable implant (12; 141; 162) than at a proximal end of the expandable implant (12; 141; 162).

11. The system (10) of any of claims 1 to 10, wherein the exterior inflatable body (105) has a thickness at the proximal portion (132) that is greater than a thickness of the exterior inflatable body (105) at the distal portion (128).

12. A delivery system for an expandable implant (12; 141; 162), the delivery system comprising:
a delivery apparatus (80) configured to deliver the expandable implant (12; 141; 162) to a location in a patient's body and including:
an elongate shaft (26), and
an inflatable device (28; 91; 97; 101) coupled to the elongate shaft (26) and including:
an interior inflatable body (14; 95) configured to inflate in response to receiving fluid, and
an exterior inflatable body (16; 81; 93; 99; 103; 105) at least partially surrounding the interior inflatable body (14; 95)and configured to inflate when the interior inflatable body (14; 95) inflates and apply an expansion force to the expandable implant (12; 141; 162) to expand the expandable implant (12; 141; 162), the exterior inflatable body (16; 81; 93; 99; 103; 105) having a distal portion, a proximal portion (46), and one or more openings at the distal portion configured to allow the fluid within the interior inflatable body (14; 95) to escape the exterior inflatable body (16; 81; 93; 99; 103; 105) when the interior inflatable body (14; 95) is punctured or bursts,
wherein the inflatable device (28; 91; 97; 101) has a first expansion rate when the interior inflatable body (14; 95) is below a threshold inflation pressure and the inflatable device has a second expansion rate when the interior inflatable body (14; 95) is above the threshold inflation pressure, the second expansion rate being lower than the first expansion rate.

13. The delivery system of claim 12, wherein the elongate shaft (26) includes a distal portion (27) and a proximal portion (29) coupled to a handle (88), and the exterior inflatable body (16; 81; 93; 99; 103; 105) and the interior inflatable body (14; 95) are each positioned on the distal portion (46) of the elongate shaft (26).

14. The delivery system of claim 12 or 13, further comprising a nose cone (86) positioned distal portion of the exterior inflatable body (16; 81; 93; 99; 103; 105) and the interior inflatable body (14; 95).

15. The delivery system of any of claims 12 to 14, further comprising an inflation lumen (40) extending along the elongate shaft (26) and configured to inflate one or more of the interior inflatable body (14; 95) or the exterior inflatable body (16; 81; 93; 99; 103; 105).

## Patentansprüche

1. System (10) zur Dilatation einer Oberfläche innerhalb des Körpers eines Patienten, wobei das System umfasst:
eine aufpumpbare Vorrichtung (28; 91; 97; 101) mit:
einen inneren aufpumpbaren Körper (14; 95), der so konfiguriert ist, dass er in Reaktion auf die Aufnahme von Fluid aufgepumpt wird; und
einen äußeren aufpumpbaren Körper (16; 81; 93; 99; 103; 105), der den inneren aufpumpbaren Körper (14; 95) wenigstens teilweise umgibt und so konfiguriert ist, dass er aufgepumpt wird, wenn der innere aufpumpbare Körper (14; 95) aufgepumpt wird, und eine Expansionskraft auf die Oberfläche ausübt, um die Oberfläche zu dehnen, wobei der äußere aufpumpbare Körper (16; 81; 93; 99; 103; 105) einen distalen Abschnitt (46), einen proximalen Abschnitt (48) und eine oder mehrere Öffnungen (32; 58) am distalen Abschnitt (46) aufweist, die so konfiguriert sind, dass das Fluid innerhalb des inneren aufpumpbaren Körpers aus dem äußeren aufpumpbaren Körper (16; 81; 93; 99; 103; 105) entweichen kann, wenn der innere aufpumpbare Körper (14; 95) durchstochen wird oder platzt,
wobei die aufpumpbare Vorrichtung (28; 91; 97; 101) eine erste Expansionsrate aufweist, wenn der innere aufpumpbare Körper (14; 95) unter einem Schwellenaufpumpdruck liegt, und die aufpumpbare Vorrichtung eine zweite Expansionsrate aufweist, wenn der innere aufpumpbare Körper (14; 95) über dem Schwellenaufpumpdruck liegt, wobei die zweite Expansionsrate kleiner als die erste Expansionsrate ist.

2. System (10) nach Anspruch 1, wobei der äußere aufpumpbare Körper (16; 81; 93; 99; 103; 105) eine geringere Nachgiebigkeit (C2) aufweist als der innere aufpumpbare Körper (14; 95), um die Expansion des inneren aufpumpbaren Körpers (14; 95) zu begrenzen.

3. System (10) nach Anspruch 1 oder 2, wobei der äußere aufpumpbare Körper (16; 81; 93; 99; 103; 105) über einen Ruhezustand hinaus expandiert, wenn der innere aufpumpbare Körper (14; 95) auf den Schwellenaufpumpdruck aufgepumpt wird.

4. System nach einem der Ansprüche 1 bis 3, wobei die erste Expansionsrate auf der Nachgiebigkeit (C1) des inneren aufpumpbaren Körpers (14; 95) basiert und die zweite Expansionsrate auf der Nachgiebigkeit (C2) des äußeren aufpumpbaren Körpers (16; 81; 93; 99; 103; 105) und der Nachgiebigkeit (c2) des inneren aufpumpbaren Körpers (14; 95) basiert.

5. System (10) nach einem der Ansprüche 1 bis 4, wobei der äußere aufpumpbare Körper (16; 81; 93; 99; 103; 105) den inneren aufpumpbaren Körper (14; 95) vor Durchstichen schützt, oder wobei der äußere aufpumpbare Körper (16; 81; 93; 99; 103; 105) so konfiguriert ist, dass er während des Aufpumpens des inneren aufpumpbaren Körpers (14; 95) Druckunterstützung bietet.

6. System (10) nach einem der Ansprüche 1 bis 5, wobei der äußere aufpumpbare Körper (16; 81; 93; 99; 103; 105) dazu konfiguriert ist, einen oder mehrere Abschnitte des inneren aufpumpbaren Körpers (14; 95) zurückzuhalten, wenn der innere aufpumpbare Körper (14; 95) durchstochen wird oder platzt.

7. System (10) nach einem der Ansprüche 1 bis 6, wobei der äußere aufpumpbare Körper (16; 81; 93; 99; 103; 105) mit einem Schaft (26) einer Zuführvorrichtung (80) am proximalen Abschnitt (48) des äußeren aufpumpbaren Körpers (16; 81; 93; 99; 103; 105) gekoppelt ist und der äußere aufpumpbare Körper (16; 81; 93; 99; 103; 105) von dem Schaft der Zuführungsvorrichtung an dem distalen Abschnitt (46) des äußeren aufpumpbaren Körpers (16; 81; 93; 99; 103; 105) getrennt ist oder mit dem Schaft (26) der Zuführungsvorrichtung (80) an dem distalen Abschnitt (46) des äußeren aufpumpbaren Körpers (16; 81; 93; 99; 103; 105) gekoppelt ist.

8. System (10) nach einem der Ansprüche 1 bis 7, wobei der innere aufpumpbare Körper (14; 95) eine erste Länge (76) aufweist und der äußere aufpumpbare Körper (16; 81; 93; 99; 103; 105) eine zweite Länge (75) aufweist, die kleiner ist als die erste Länge (76).

9. System (10) nach einem der Ansprüche 1 bis 8, wobei die Oberfläche eine Innenfläche (19) eines expandierbaren Implantats (12; 141; 162) umfasst.

10. System (10) nach einem der Ansprüche 1 bis 9, wobei ein Durchmesser (136) des inneren aufpumpbaren Körpers (95) an einem distalen Abschnitt (46) des inneren aufpumpbaren Körpers (14; 95) größer ist als ein Durchmesser (138) des äußeren aufpumpbaren Körpers (16; 81; 93; 99; 103; 105) am proximalen Abschnitt (46) des äußeren aufpumpbaren Körpers (16; 81; 93; 99; 103; 105), und wobei die Oberfläche eine innere Oberfläche (19) eines expandierbaren Implantats umfasst, die an einem distalen Ende des expandierbaren Implantats (12; 141; 162) weiter ist als an einem proximalen Ende des expandierbaren Implantats (12; 141; 162).

11. System (10) nach einem der Ansprüche 1 bis 10, wobei der äußere aufpumpbare Körper (105) am proximalen Abschnitt (132) eine Dicke aufweist, die größer ist als eine Dicke des äußeren aufpumpbaren Körpers (105) am distalen Abschnitt (128).

12. Zuführsystem für ein expandierbares Implantat (12; 141; 162), wobei das Zuführsystem umfasst:
eine Zuführvorrichtung (80), die dazu konfiguriert ist, das expandierbare Implantat (12; 141; 162) an eine Stelle im Körper eines Patienten zuzuführen, und die umfasst:
einen langgestreckten Schaft (26), und
eine aufpumpbare Vorrichtung (28; 91; 97; 101), die mit dem langgestreckten Schaft (26) gekoppelt ist und aufweist:
einen inneren aufpumpbaren Körper (14; 95), der so konfiguriert ist, dass er in Reaktion auf die Aufnahme von Fluid aufgepumpt wird, und
einen äußeren aufpumpbaren Körper (16; 81; 93; 99; 103; 105), der den inneren aufpumpbaren Körper (14; 95) wenigstens teilweise umgibt und so konfiguriert ist, dass er aufgepumpt wird, wenn der innere aufpumpbare Körper (14; 95) aufgepumpt wird, und eine Expansionskraft auf das expandierbare Implantat (12; 141; 162) ausübt, um das expandierbare Implantat (12; 141; 162) zu expandieren, wobei der äußere aufpumpbare Körper (16; 81; 93; 99; 103; 105) einen distalen Abschnitt, einen proximalen Abschnitt (46) und eine oder mehrere Öffnungen am distalen Abschnitt aufweist, die so konfiguriert sind, dass das Fluid innerhalb des inneren aufpumpbaren Körpers (14; 95) aus dem äußeren aufpumpbaren Körper (16; 81; 93; 99; 103; 105) entweichen kann, wenn der innere aufpumpbare Körper (14; 95) durchstochen wird oder platzt,
wobei die aufpumpbare Vorrichtung (28; 91; 97; 101) eine erste Expansionsrate aufweist, wenn der innere aufpumpbare Körper (14; 95) unter einem Schwellenaufpumpdruck liegt, und die aufpumpbare Vorrichtung eine zweite Expansionsrate aufweist, wenn der innere aufpumpbare Körper (14; 95) über dem Schwellenaufpumpdruck liegt, wobei die zweite Expansionsrate kleiner als die erste Expansionsrate ist.

13. Zuführsystem nach Anspruch 12, wobei der langgestreckte Schaft (26) einen distalen Abschnitt (27) und einen proximalen Abschnitt (29) aufweist, die mit einem Griff (88) gekoppelt sind, und der äußere aufpumpbare Körper (16; 81; 93; 99; 103; 105) und der innere aufpumpbare Körper (14; 95) jeweils auf dem distalen Abschnitt (46) des langgestreckten Schafts (26) angeordnet sind.

14. Zuführsystem nach Anspruch 12 oder 13, das ferner einen Nasenkonus (86) umfasst, der am distalen Abschnitt des äußeren aufpumpbaren Körpers (16; 81; 93; 99; 103; 105) und des inneren aufpumpbaren Körpers (14; 95) angeordnet ist.

15. Zuführsystem nach einem der Ansprüche 12 bis 14, das ferner ein Aufpumplumen (40) umfasst, das sich entlang des langgestreckten Schafts (26) erstreckt und dazu konfiguriert ist, einen oder mehrere von dem inneren aufpumpbaren Körper (14; 95) oder dem äußeren aufpumpbaren Körper (16; 81; 93; 99; 103; 105) aufzupumpen.

## Revendications

1. Système (10) pour la dilatation d'une surface à l'intérieur du corps d'un patient,
le système comprenant :
un dispositif gonflable (28 ; 91 ; 97 ; 101) présentant :
un corps gonflable intérieur (14 ; 95) conçu pour se gonfler en réponse à la réception d'un fluide ; et
un corps gonflable extérieur (16 ; 81 ; 93 ; 99 ; 103 ; 105) entourant au moins partiellement le corps gonflable intérieur (14 ; 95) et conçu pour se gonfler lorsque le corps gonflable intérieur (14 ; 95) se gonfle et pour appliquer une force d'expansion à la surface en vue de dilater la surface, le corps gonflable extérieur (16 ; 81 ; 93 ; 99 ; 103 ; 105) présentant une partie distale (46), une partie proximale (48) et une ou plusieurs ouvertures (32 ; 58) au niveau de la partie distale (46) conçues pour permettre au fluide à l'intérieur du corps gonflable intérieur de s'échapper du corps gonflable extérieur (16 ; 81 ; 93 ; 99 ; 103 ; 105) lorsque le corps gonflable intérieur (14 ; 95) est perforé ou éclate,
le dispositif gonflable (28 ; 91 ; 97 ; 101) présentant un premier taux d'expansion lorsque le corps gonflable intérieur (14 ; 95) est à une pression inférieure à une pression de gonflage seuil et le dispositif gonflable présentant un second taux d'expansion lorsque le corps gonflable intérieur (14 ; 95) est à une pression supérieure à la pression de gonflage seuil, le second taux d'expansion étant inférieur au premier taux d'expansion.

2. Système (10) selon la revendication 1, le corps gonflable extérieur (16 ; 81 ; 93 ; 99 ; 103 ; 105) présentant une compliance inférieure (C2) à celle du corps gonflable intérieur (14 ; 95) afin de limiter l'expansion du corps gonflable intérieur (14 ; 95).

3. Système (10) selon la revendication 1 ou 2, le corps gonflable extérieur (16 ; 81 ; 93 ; 99 ; 103 ; 105) s'étendant au-delà d'un état de repos lorsque le corps gonflable intérieur (14 ; 95) est gonflé à la pression de gonflage seuil.

4. Système selon l'une quelconque des revendications 1 à 3, le premier taux d'expansion étant basé sur la compliance (C1) du corps gonflable intérieur (14 ; 95) et le second taux d'expansion étant basé sur la compliance (C2) du corps gonflable extérieur (16 ; 81 ; 93 ; 99 ; 103 ; 105) et sur la compliance (c2) du corps gonflable intérieur (14 ; 95).

5. Système (10) selon l'une quelconque des revendications 1 à 4, le corps gonflable extérieur (16 ; 81 ; 93 ; 99 ; 103 ; 105) protégeant le corps gonflable intérieur (14 ; 95) contre une perforation ou
le corps gonflable extérieur (16 ; 81 ; 93 ; 99 ; 103 ; 105) étant conçu pour fournir un support de pression pendant le gonflage du corps gonflable intérieur (14 ; 95).

6. Système (10) selon l'une quelconque des revendications 1 à 5, le corps gonflable extérieur (16 ; 81 ; 93 ; 99 ; 103 ; 105) étant conçu pour retenir une ou plusieurs parties du corps gonflable intérieur (14 ; 95) lorsque le corps gonflable intérieur (14 ; 95) est perforé ou éclate.

7. Système (10) selon l'une quelconque des revendications 1 à 6, le corps gonflable extérieur (16 ; 81 ; 93 ; 99 ; 103 ; 105) étant accouplé à une tige (26) d'un appareil de pose (80) au niveau de la partie proximale (48) du corps gonflable extérieur (16 ; 81 ; 93 ; 99 ; 103 ; 105) et le corps gonflable extérieur (16 ; 81 ; 93 ; 99 ; 103 ; 105) étant détaché de la tige de l'appareil de pose au niveau de la partie distale (46) du corps gonflable extérieur (16 ; 81 ; 93 ; 99 ; 103 ; 105) ou étant accouplé à la tige (26) de l'appareil de pose (80) au niveau de la partie distale (46) du corps gonflable extérieur (16 ; 81 ; 93 ; 99 ; 103 ; 105).

8. Système (10) selon l'une quelconque des revendications 1 à 7, le corps gonflable intérieur (14 ; 95) présentant une première longueur (76) et le corps gonflable extérieur (16 ; 81 ; 93 ; 99 ; 103 ; 105) présentant une seconde longueur (75) qui est inférieure à la première longueur (76).

9. Système (10) selon l'une quelconque des revendications 1 à 8, la surface comprenant une surface intérieure (19) d'un implant déployable (12 ; 141 ; 162).

10. Système (10) selon l'une quelconque des revendications 1 à 9, un diamètre (136) du corps gonflable intérieur (95) au niveau d'une partie distale (46) du corps gonflable intérieur (14 ; 95) étant supérieur à un diamètre (138) du corps gonflable extérieur (16 ; 81 ; 93 ; 99 ; 103 ; 105) au niveau de la partie proximale (46) du corps gonflable extérieur (16 ; 81 ; 93 ; 99 ; 103 ; 105) et la surface comprenant une surface intérieure (19) d'un implant déployable plus large au niveau d'une extrémité distale de l'implant déployable (12 ; 141 ; 162) qu'au niveau d'une extrémité proximale de l'implant déployable (12 ; 141 ; 162).

11. Système (10) selon l'une quelconque des revendications 1 à 10, le corps gonflable extérieur (105) présentant une épaisseur au niveau de la partie proximale (132) qui est supérieure à une épaisseur du corps gonflable extérieur (105) au niveau de la partie distale (128).

12. Système de pose pour un implant déployable (12 ; 141 ; 162), le système de pose comprenant :
un appareil de pose (80) conçu pour poser l'implant déployable (12 ; 141 ; 162) en un emplacement dans le corps d'un patient et comprenant :
une tige allongée (26) et
un dispositif gonflable (28 ; 91 ; 97 ; 101) accouplé à la tige allongée (26) et comprenant :
un corps gonflable intérieur (14 ; 95) conçu pour se gonfler en réponse à la réception d'un fluide ; et
un corps gonflable extérieur (16 ; 81 ; 93 ; 99 ; 103 ; 105) entourant au moins partiellement le corps gonflable intérieur (14 ; 95) et conçu pour se gonfler lorsque le corps gonflable intérieur (14 ; 95) se gonfle et pour appliquer une force d'expansion à l'implant déployable (12 ; 141 ; 162) afin de déployer l'implant déployable (12 ; 141 ; 162), le corps gonflable extérieur (16 ; 81 ; 93 ; 99 ; 103 ; 105) présentant une partie distale, une partie proximale (46) et une ou plusieurs ouvertures au niveau de la partie distale conçues pour permettre au fluide à l'intérieur du corps gonflable intérieur (14 ; 95) de s'échapper du corps gonflable extérieur (16 ; 81 ; 93 ; 99 ; 103 ; 105) lorsque le corps gonflable intérieur (14 ; 95) est perforé ou éclate,
le dispositif gonflable (28 ; 91 ; 97 ; 101) présentant un premier taux d'expansion lorsque le corps gonflable intérieur (14 ; 95) est à une pression inférieure à une pression de gonflage seuil et le dispositif gonflable présentant un second taux d'expansion lorsque le corps gonflable intérieur (14 ; 95) est à une pression supérieure à la pression de gonflage seuil, le second taux d'expansion étant inférieur au premier taux d'expansion.

13. Système de pose selon la revendication 12, la tige allongée (26) comprenant une partie distale (27) et une partie proximale (29) accouplée à une poignée (88) et le corps gonflable extérieur (16 ; 81 ; 93 ; 99 ; 103 ; 105) et le corps gonflable intérieur (14 ; 95) étant chacun positionnés sur la partie distale (46) de la tige allongée (26).

14. Système de pose selon la revendication 12 ou 13, comprenant en outre une pointe avant (86) positionnée distalement par rapport au corps gonflable extérieur (16 ; 81 ; 93 ; 99 ; 103 ; 105) et au corps gonflable intérieur (14 ; 95).

15. Système de pose selon l'une quelconque des revendications 12 à 14, comprenant en outre une lumière de gonflage (40) s'étendant le long de la tige allongée (26) et conçue pour gonfler un ou plusieurs corps parmi le corps gonflable intérieur (14 ; 95) ou le corps gonflable extérieur (16 ; 81 ; 93 ; 99 ; 103 ; 105).
